(19) Europäisches Patentamt
European Patent Office
Office européen des brevets

(11) EP 2 874 711 B1

(12) # FASCICULE DE BREVET EUROPEEN

(45) Date de publication et mention de la délivrance du brevet:
**13.03.2019 Bulletin 2019/11**

(21) Numéro de dépôt: **13734771.2**

(22) Date de dépôt: **08.07.2013**

(51) Int Cl.:
*A61Q 19/00* (2006.01) *A61Q 17/00* (2006.01)
*A61P 17/10* (2006.01) *A61K 8/27* (2006.01)
*A61K 8/46* (2006.01)

(86) Numéro de dépôt international:
**PCT/EP2013/064391**

(87) Numéro de publication internationale:
**WO 2014/009315 (16.01.2014 Gazette 2014/03)**

(54) **UTILISATION DE COCETH SULFATE DE ZINC COMME AGENT ANTIBACTERIEN VIS A VIS DE PROPIONIBACTERIUM ACNES**

VERWENDUNG VON ZINC COCETH SULFATE ALS ANTIBAKTERIELLES MITTEL GEGEN PROPIONIBACTERIUM ACNES

USE OF ZINC COCETH SULFATE AS AN ANTIBACTERIAL AGENT AGAINST PROPIONIBACTERIUM ACNES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.07.2012 FR 1256606**

(43) Date de publication de la demande:
**27.05.2015 Bulletin 2015/22**

(73) Titulaire: **Pierre Fabre Dermo-Cosmétique**
**92100 Boulogne-Billancourt (FR)**

(72) Inventeur: **BACO, David**
**F-31860 Labarthe sur Leze (FR)**

(74) Mandataire: **Regimbeau**
**20, rue de Chazelles**
**75847 Paris Cedex 17 (FR)**

(56) Documents cités:
**EP-A1- 0 068 055** **EP-A2- 1 074 247**
**WO-A1-00/16791** **WO-A2-2012/163928**

- **ANONYMOUS: "ZETESOL ZN An alkyl ether sulfate with surprising applications", Zschimmer & Schwarz Italiana S.p.A. , 2003, pages 1-28, XP002691939, Extrait de l'Internet: URL:http://www.erwebhosting.it/zsi/repository/Zetesol%20ZN%20-%20An%20alkyl%20ether% 20sulfate%20with%20surprising%20application.pdf [extrait le 2013-02-10]**
- **"Sodium lauroyl methyl isethionate" In: "International Cosmetic Ingredient Dictionary and Handbook Thirteenth Edition Volume 2", 1 janvier 2010 (2010-01-01), Personal Care Products Council, Washington, XP55007319, ISBN: 978-1-88-262147-7 page 2863, chapitre "SODIUM LAUROYL METHYL ISETHIONATE", paragraphe "Reported Function"**
- **BIGOTTI C ET AL: "Zinc and its derivatives: their applications in cosmetic", JOURNAL OF APPLIED COSMETOLOGY, INTERNATIONAL EDIEMME, ROME, IT, 1 janvier 2005 (2005-01-01), pages 139-147, XP009166992, ISSN: 0392-8543**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

Le domaine de la présente invention concerne principalement les nouvelles propriétés antibactériennes du zinc coceth sulfate vis à vis de la bactérie *Propionibacterium acnes* ; et ses applications dans les domaines de la cosmétique et de la dermatologie pour le traitement de l'acné et des désordres cutanés liés à *Propionibacterium acnes.*

Le sébum est la sécrétion par les glandes sébacées de la peau d'un film lipidique qui sert à la protéger et, mélangée à la sueur, protège la peau du dessèchement.

Le sébum permet l'imperméabilisation de la peau, participe au développement de la structure épidermique. Il la protège des microbes en l'acidifiant (présence d'acide lactique et d'acides gras) et assure une certaine imperméabilité. Il permet à la peau d'être souple et participe au développement de l'épiderme.
Le sébum arrive normalement à la surface de la peau par les pores des follicules pileux.

Au cours de l'acné, l'excès de sébum dans l'infundibulum pilaire représente un environnement propice pour la colonisation de *Propionibacterium acnes.* L'apparition des lésions dans l'acné dépend d'une réponse pro-inflammatoire trop intense, via les récepteurs de l'immunité innée, vis-à-vis d'une densité trop importante en *Propionibacterium acnes.*

Ces bactéries possèdent en effet la particularité de métaboliser les triglycérides du sébum en libérant des acides gras qui provoquent l'inflammation des tissus.

De façon inattendue et surprenante, la demanderesse a mis en évidence que le coceth sulfate de zinc peut être utilisé comme agent antibactérien vis à vis de *Propionibacterium acnes.*

Le coceth sulfate de zinc (Zetesol Zn® de la société Zchimmer&Schwarz) est un tensioactif anionique dont la tolérance est très supérieure au lauryl ether sulfate de sodium classique en raison de la deuxième chaîne laurique fixée sur le métal. La présence moléculaire du zinc lui confère une activité très intéressante sur les germes des peaux grasses et permet de limiter fortement les agents conservateurs (concentration entre 2% et 7%) .

Le Coceth sulfate de zinc est un agent tensioactif de nouvelle génération, associant un alkyléthersulfate à du zinc (double chaîne lipophile de Coprah / zinc).

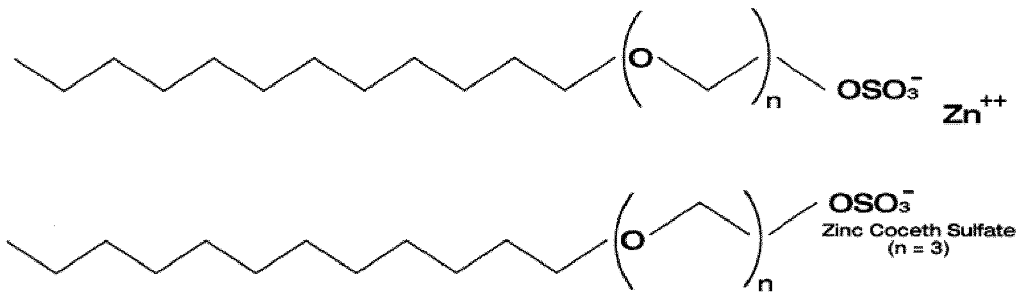

Zinc Coceth Sulfate
(n = 3)

Les alkyléthersulfates sont les tensioactifs les plus largement employés dans les produits cosmétiques et de toilette. Associés en mélanges binaires ou ternaires, ils sont des moussants appréciables (non affectés par l'eau dure) et ont un excellent pouvoir nettoyant.

Ils offrent une bonne tolérance cutanée et oculaire qui peut être encore améliorée selon le cation utilisé pour les neutraliser.

L'étude menée sur le coceth sulfate de zinc a permis de démontrer non seulement qu'il développait de bonnes propriétés tensioactives associées à une excellente tolérance locale, mais aussi qu'il était doté d'autres propriétés intéressantes.
Comme les alkyléthersulfates, le coceth sulfate de zinc a confirmé ses bonnes propriétés nettoyantes: pouvoir moussant satisfaisant, obtention d'une mousse douce et crémeuse, agréable à l'utilisation.

Sa compatibilité avec la peau est apparue cependant étonnante, son potentiel d'agression cutanée étant largement inférieur à celui des alkyléthersulfates et vraisemblablement lié à sa double chaîne lipophile.

Son pouvoir nettoyant s'est en outre montré indifférent à la dureté de l'eau.

Le coceth sulfate de zinc a affiché un pH acide de 4.5, proche du pH cutané de l'ordre de 5.5.

Il n'agresse donc pas la peau dont l'acidité est une des caractéristiques importantes chez l'adulte comme chez le jeune enfant.

Le coceth sulfate de zinc s'est révélé dénué de tout potentiel irritant et sensibilisant.

Le coceth sulfate de zinc a également fait la preuve de sa bonne tolérance sur muqueuse.

La démonstration en a été faite à l'aide du test de Het Cam dont le principe est basé sur l'observation des effets irritants d'un produit pouvant survenir dans les cinq minutes suivant son dépôt sur la membrane chorio-allantoïdienne d'un oeuf de poule embryonné, au dixième jour d'incubation. Le Coceth de zinc a été expérimenté en solution à 1%.

L'étude bactériologique a été réalisée sur des solutions aqueuses de Coceth sulfate de zinc à 10% et 20%

avec un pH final compris entre 5 et 6.

L'étude bactériologique a été réalisée sur *Propionibacterium acnes,* bactérie responsables des peaux acnéiques : une étude menée au laboratoire de microbiologie a démontré cette efficacité de bactéricide. Cette étude a été confirmée sur le produit fini.

La présente invention vise donc une composition d'hygiène de la peau comprenant du coceth sulfate de zinc en tant qu'agent antibactérien vis-à-vis de Propionibacterium acnes en association avec le sodium lauroyl méthyl iséthionate. Selon une autre caractéristique de l'invention, ladite composition est utile en tant qu'agent antibactérien vis à vis de *Propionibacterium acnes.* Plus particulièrement, ladite composition est destinée au traitement de l'acné.

Un autre aspect de la présente invention concerne l'utilisation non thérapeutique d'une composition comprenant du coceth sulfate de zinc comme agent antibactérien vis à vis de *Propionibacterium acnes.*

Contrairement à certaines compositions de nettoyage de la peau de type analogue qui préconisent la présence obligatoire d'un sel ou dérivé additionnel d'acide glycyrrhizique ou d'acide glycyrrhétinique, la composition objet de la présente demande est expressément dépourvue de tels sels ou dérivés additionnels.

De préférence, l'utilisation cosmétique est destinée au traitement et/ou à l'hygiène des peaux acnéiques et/ou des peaux grasses à tendance acnéique.

L'hygiène des peaux acnéiques et/ou des peaux grasses à tendance acnéique nécessite l'usage de produits adaptés à la fragilité tissulaire et à la flore microbienne accompagnant la pathologie.

De préférence, dans le cadre du développement d'un produit nettoyant pour les peaux acnéiques et/ou les peaux grasses à tendance acnéique, il est nécessaire de répondre à plusieurs critères :

- Bon pouvoir délipidant des corps gras sébaciques
- Bonne tolérance tensioactive de façon à ne pas stimuler par effet rebond la glande sébacée
- Réguler la fonction sébacée
- Bloquer la croissance bactérienne associée.

Un autre objet de la présente invention concerne une composition, de préférence une composition nettoyante pour les peaux acnéiques et/ou à tendance acnéiques comprenant du coceth sulfate de zinc en tant qu'agent antibactérien vis à vis de *Propionibacterium acnes* en association avec

- le tensioactif anionique sodium lauroyl méthyl isethionate ;
- et/ou un autre agent antibactérien choisi parmi l'extrait myrte et plus particulièrement l'extrait de myrte tel que décrit dans EP1112079, le polyglyceryl-3 monocaprylate, le glyceryl caprate, le bromure de cetrimonium,
- et/ou un agent anti séborrhée choisi parmi le glycéryl laurate tel que décrit dans WO2011/073370, extrait de sabal, huile de pepin de courge, extrait d'ortie dioique.

Dans un mode de réalisation particulier de l'invention, la base excipiendaire de la composition selon l'invention, associe en mélange essentiellement binaire des surfactants anioniques dont le coceth sulfate de zinc éventuellement associé avec le sodium lauroyl méthyl iséthionate ; et des surfactants non ioniques dans le rapport 80/20 exprimés en matière active. La présence d'un amphotère à faible concentration permet en outre de stabiliser la viscosité finale.

Le sodium lauroyl méthyl iséthionate (Iselux®, Innospec) est dans la classe des tensioactifs anionique l'un des mieux toléré. Son usage en dermopain depuis de nombreuses années, confirme son excellente biocompatibilité cutanée, tant dans le domaine pédiatrique que celui de l'atopie (concentration entre 5% et 10%).

Dans un autre mode de réalisation préférée de l'invention, le coceth sulfate de zinc en tant qu'agent antibactérien vis à vis de *P. acnes* sera utilisé en association avec un autre actif :

- antibactérien tel que l'extrait de myrte et préférentiellement l'extrait de myrte décrit dans EP1112079, le polyglycéryl-3 monocaprylate, le glycéryl caprate, le bromure de cétrimonium
- ou d'action complémentaire sur la pathologie de l'acné tel que le glycéryl laurate, extrait de sabal, huile de pepin de courge, extrait d'ortie dioique.

Préférentiellement, les tensioactifs anioniques coceth sulfate de zinc et le sodium lauroyl méthyl iséthionate tel que l'Iselux® seront associés dans une composition nettoyante désinfectante de la peau.

En outre, il est connu que les agents de surface non ioniques bipolaires apportent une extrême douceur au produit en raison de leur structure moléculaire associant leur chaîne grasse à une chaîne oxyethylénée sous forme d'ether :

- le polysorbate 20 associant le sorbitol cyclisé à une chaîne laurique éthoxylée
- l'huile de ricin hydrogénée ethoxylée (40EO)

- le cétéareth-60 myristyl glycol dont la chaîne grasse assure une protection cutanée de surface.

Tamponné à pH=5 par l'EDTA 2Na et l'acide citrique, la formule est parfaitement adaptée au pH cutané des peaux grasses. Le pH acide participe à l'activité antibactérienne et anti-5$\alpha$réductase du glycéryl laurate associé au gluconate de zinc anti-inflammatoire.
Préférentiellement, les compositions selon l'invention seront administrées par voie topique.
Plus préférentiellement, les compositions selon l'invention se présentent sous forme de gel nettoyant liquide, de foamer ou d'aérosol.
C'est enfin un autre objet de la présente invention que de fournir un procédé cosmétique pour assainir (et/ou nettoyer) les peaux acnéiques et/ou les peaux grasses à tendance acnéique caractérisé en ce que l'on applique sur lesdites peaux une composition comprenant du coceth sulfate de zinc.
L'invention pourra être mieux comprise à l'aide des exemples non limitatifs qui suivent et qui constituent des modes de réalisation particuliers des compositions cosmétiques et/ou dermatologiques selon l'invention.

EXEMPLES DE COMPOSITION SELON L'INVENTION

Exemple 1 :

Sodium Lauroyl méthyl iséthionate (Iselux®, Innospec) de 5 à 20% Zinc coceth sulfate (Zetesol Zn®, Zchimmer&Schwarz) de 5 à 20% Disodium cocoamphoacétate de 3 à 10%
Polysorbate 20 de 0.5 à 4%
Cétéareth-60 myristyl glycol de 0.5 à 1.5%
Huile de ricin hydrogénée-éthoxylée de 0.5% à 2%
Glyceryl laurate de 0.1 à 1%
Zinc gluconate de 0.1 à 0.5%
Acide citrique / EDTA 2Na qsp pH=5
EVALUATION DE L'ACTIVITE BACTERICIDE du ZETESOL Zn® vis à vis de *P. acnes.*
On évalue la réduction dans le temps du log10 du nombre de germes par rapport au nombre de germes initial(paramètre log R).
La souche testée est *Propionibacterium acnes* ATCC6919.
Le Zetesol Zn® a été testé à la concentration de 20% (soit 5% de matière active coceth sulfate de zinc).

Méthode

- mise en contact du produit à tester et des suspensions de *P. acnes* titrées à environ $10^6$ UFC/ml pendant 1, 5, 10 et 15 minutes.
- Dénombrement après chaque temps de contact par inclusion de 1ml de l'échantillon dans une gélose de culture (Columbia)
- Incubation 72 heures à 36°C en anaérobiose
- Dénombrement des bactéries résiduelles par comptage direct.

Calcul de la chute de log10 :
La numération des boîtes est effectuée et on rapporte ce résultat à un nombre d'UFC / ml.
Le calcul de la chute de Log10 du nombre d'UFC /ml est réalisé suivant la formule ci-dessous :

$$\text{Réduction logarithmique} = \text{LOG}_{10}\ (\text{nombre d'UFC présentes à TO / nombre d'UFC restantes à Tx})$$

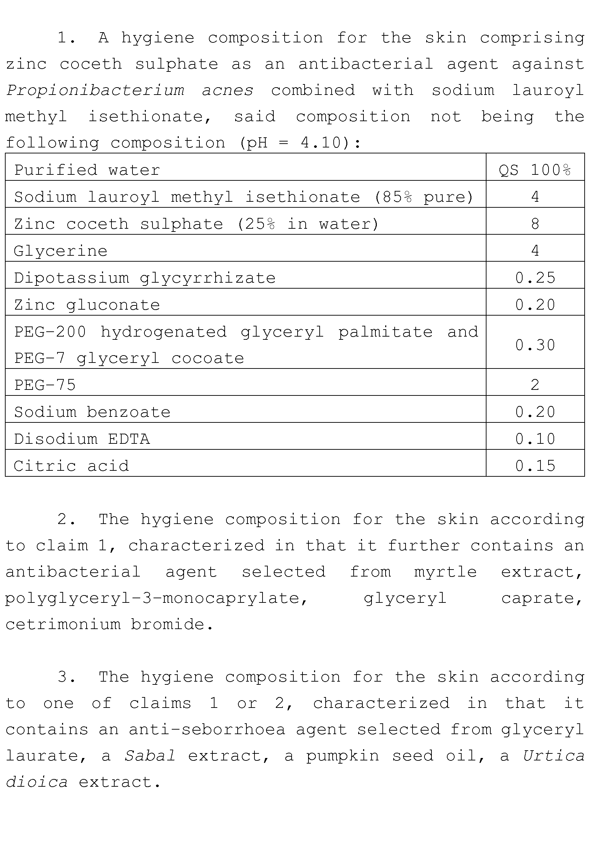

Les résultats indiqués dans le tableau 1 sont la moyenne de deux essais indépendants (excepté au temps 10min où 1 seul essai a été réalisé). Voir également figure 1.

Tableau 1

| Concentration du Zetesol Zn® | Réduction Log en 1 minute | Réduction Log en 5 minutes | Réduction Log en 10 minutes | Réduction Log en 15 minutes |
|---|---|---|---|---|
| 20% | 0.63 | 2.06 | 3.65 | > 5.04 |

On observe une bonne activité bactéricide dès 1 minute de contact qui est proche du temps d'utilisation d'un produit rincé.
L'activité est optimale à 15 minutes (seuil maximal visualisable atteint).

Exemple 2 :

Un autre exemple de composition selon l'invention a été soumis à la même évaluation de l'activité bactéricide conformément au même protocole rappelé ci-dessus.
Ladite composition testée répond à la formulation suivante :

- Zetesol Zn (concentration 20 %, soit 5% de matière active coceth sulfate de zinc)
- Iselux (concentration 10 %, soit 8% de matière active sodium lauroyl methyl iséthionate)
- Ceteareth-60 myristyl glycol-épaississant
- Cocoamphoacetate Na - tensioactif amphotère
- Capryl glycol
- Acide lactique
- NaOH
- Qs eau

Les résultats obtenus sont rappelés dans le Tableau 2 ci-dessous :

Tableau 2

| | Réduction Log en 1 minute | Réduction Log en 5 minutes | Réduction Log en 10 minutes | Réduction Log en 15 minutes |
|---|---|---|---|---|
| Zetesol Zn 20 % + Iselyx 10 % | 1,9 | 2,6 | 3,5 | > 4.2 |

On observe une réduction de 1,9 log en 1 minute sur les souches de *Propionibacterium acnes* ATCC6919. Il apparaît ainsi que l'ajout de sodium lauroyl méthyl iséthionate a permis d'améliorer significativement l'activité bactéricide du coceth sulfate de zinc vis-à-vis de P. *acnes.*
EVALUATION DE L'ACTIVITE BACTERICIDE du produit formulé selon l'exemple 1 vis à vis de *P. acnes.*
Le même protocole que ci-dessus est utilisé.

Produit testé :

- composition selon l'exemple 1 diluée à 20% dans l'eau distillée stérile = dilution d'utilisation d'un produit rincé.

Les souches testées sont

- *Propionibacterium acnes* ATCC6919.
- *Propionibacterium acnes* Sauvage (souche érythromycine résistant R)
- *Propionibacterium acnes* Sauvage (souche érythromycine sensible S)

Les résultats indiqués dans le tableau 3 ci-dessous sont la moyenne de 2 essais indépendants. Voir également la figure 2.

Tableau 3

| Germes | Réduction Log en 1 minute | Réduction Log en 5 minutes | Réduction Log en 15 minutes |
|---|---|---|---|
| Propionibacterium acnes ATCC6919 | 2.35 | 3.85 | 5.25 |
| Propionibacterium acnes Sauvage (ery R) | 2.15 | 3.6 | 5 |
| Propionibacterium acnes Sauvage (ery S) | 4.75 | > 5.6 | > 5.6 |

Même diluée à 20%, la composition conserve une bonne activité.

## Revendications

**1.** Composition d'hygiène de la peau comprenant du coceth sulfate de zinc en tant qu'agent antibactérien vis-à-vis de *Propionibacterium acnes* en association avec le sodium lauroyl méthyl iséthionate, ladite composition n'étant pas la composition suivante (pH = 4,10):

| | |
|---|---|
| Eau purifiée | QSP 100% |
| Sodium lauroyl méthyl iséthionate (pur à 85%) | 4 |
| Coceth sulfate de zinc (25% dans l'eau) | 8 |
| Glycérine | 4 |
| Glycyrrhizate dipotassique | 0,25 |
| Gluconate de zinc | 0,20 |
| PEG-200 palmitate de glycéryle hydrogéné et PEG-7 cocoate de glycéryle | 0,30 |
| PEG-75 | 2 |
| Benzoate de sodium | 0,20 |
| EDTA disodique | 0,10 |
| Acide citrique | 0,15 |

**2.** Composition d'hygiène de la peau selon la revendication 1, **caractérisée en ce qu'**elle contient en outre un agent antibactérien choisi parmi l'extrait de myrte, le polyglycéryl-3 monocaprylate, le glycéryl caprate, le bromure de cétrimonium.

**3.** Composition d'hygiène de la peau selon l'une des revendications 1 ou 2, **caractérisée en ce qu'**elle contient un agent anti-séborrhée choisi parmi le glycéryl laurate, un extrait de sabal, une huile de pépin de courge, un extrait d'ortie dioïque.

## Patentansprüche

**1.** Hauthygienezusammensetzung, die Zinc Coceth Sulfate als antibakterielles Mittel gegen Propionibakterium Acnes in Verbindung mit Natriumlauroyl-Methylisethionat umfasst, wobei die Zusammensetzung nicht die folgende Zusammensetzung (pH = 4,10) ist:

| | |
|---|---|
| Gereinigtes Waser | QSP 100% |
| Natriumlauroyl-Methylisethionat (zu 85% rein) | 4 |

(fortgesetzt)

| | |
|---|---|
| Zinc Coceth Sulfate (25% in Wasser) | 8 |
| Glyzerin | 4 |
| Dikaliumglycyrrhizat | 0,25 |
| Zinkgluconat | 0,20 |
| PEG-200 hydriertes Glycerylpalmitat und PEG-7 Glyceryl-Cocoate | 0,30 |
| PEG-75 | 2 |
| Natriumbenzoat | 0,20 |
| Dinatrium-EDTA | 0,10 |
| Zitronensäure | 0,15 |

2. Hauthygienezusammensetzung nach Anspruch 1, **dadurch gekennzeichnet, dass** sie ferner ein antibakterielles Mittel enthält, das unter Myrtenextrakt, Polyglyceryl-3-Monocaprylat, Glyceryl-Caprate, Cetrimoniumbromid ausgewählt ist.

3. Hauthygienezusammensetzung nach einem der Ansprüche 1 oder 2, **dadurch gekennzeichnet, dass** sie ein Anti-Seborrhoemittel enthält, das unter Glyceryllaurat, einem Sabalextrakt, einem Kürbiskernöl, einem Brennesselextrakt ausgewählt ist.

**Claims**

1. A hygiene composition for the skin comprising zinc coceth sulphate as an antibacterial agent against *Propionibacterium acnes* combined with sodium lauroyl methyl isethionate, said composition not being the following composition (pH = 4.10):

| | |
|---|---|
| Purified water | QS 100% |
| Sodium lauroyl methyl isethionate (85% pure) | 4 |
| Zinc coceth sulphate (25% in water) | 8 |
| Glycerine | 4 |
| Dipotassium glycyrrhizate | 0.25 |
| Zinc gluconate | 0.20 |
| PEG-200 hydrogenated glyceryl palmitate and PEG-7 glyceryl cocoate | 0.30 |
| PEG-75 | 2 |
| Sodium benzoate | 0.20 |
| Disodium EDTA | 0.10 |
| Citric acid | 0.15 |

2. The hygiene composition for the skin according to claim 1, **characterized in that** it further contains an antibacterial agent selected from myrtle extract, polyglyceryl-3-monocaprylate, glyceryl caprate, cetrimonium bromide.

3. The hygiene composition for the skin according to one of claims 1 or 2, **characterized in that** it contains an anti-seborrhoea agent selected from glyceryl laurate, a *Sabal* extract, a pumpkin seed oil, a *Urtica dioica* extract.

1. A hygiene composition for the skin comprising zinc coceth sulphate as an antibacterial agent against *Propionibacterium acnes* combined with sodium lauroyl methyl isethionate, said composition not being the following composition (pH = 4.10):

| | |
|---|---|
| Purified water | QS 100% |
| Sodium lauroyl methyl isethionate (85% pure) | 4 |
| Zinc coceth sulphate (25% in water) | 8 |
| Glycerine | 4 |
| Dipotassium glycyrrhizate | 0.25 |
| Zinc gluconate | 0.20 |
| PEG-200 hydrogenated glyceryl palmitate and PEG-7 glyceryl cocoate | 0.30 |
| PEG-75 | 2 |
| Sodium benzoate | 0.20 |
| Disodium EDTA | 0.10 |
| Citric acid | 0.15 |

2. The hygiene composition for the skin according to claim 1, characterized in that it further contains an antibacterial agent selected from myrtle extract, polyglyceryl-3-monocaprylate, glyceryl caprate, cetrimonium bromide.

3. The hygiene composition for the skin according to one of claims 1 or 2, characterized in that it contains an anti-seborrhoea agent selected from glyceryl laurate, a *Sabal* extract, a pumpkin seed oil, a *Urtica dioica* extract.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1112079 A
- WO 2011073370 A